# EUROPÄISCHE PATENTSCHRIFT

(11) **EP 2 452 655 B1**
(45) Veröffentlichungstag und Bekanntmachung des Hinweises auf die Patenterteilung: **16.04.2014**
(21) Anmeldenummer: 10014658.8
(22) Anmeldetag: 16.11.2010
(51) Int. Cl.: A61F 5/37

(54) **Fixbandage zur Fixierung eines Patienten**
Fixing bandage for fixing a patient
Bandage fixe pour la fixation d'un patient

(43) Veröffentlichungstag der Anmeldung: 16.05.2012
(73) Patentinhaber: Wysozki, Roman, 22763 Hamburg (DE)
(72) Erfinder: Wysozki, Roman, 22763 Hamburg (DE)
(74) Vertreter: Hauck Patent- und Rechtsanwälte

(56) Entgegenhaltungen:
- DE-U1-202006 009 390
- JP-A- 60 055 944

## Beschreibung

Die Erfinding betrifft eine Fixierbandage zur Fixierung eines Patienten auf einem Bett oder einem sonstigen Lager.

Eine zu diesem Zweck bekannte Fixierbandage weist einen flach auf das Bett aufzulegenden Bettgurt auf, der zur Befestigung an einem Bettgestell des Betts an jedem seiner beiden Enden eine Betthalterung aufweist. Die Betthalterungen werden jeweils schlaufenförmig um einen Holm des Bettgestells herumgeschlungen und mit einer geeigneten Verriegelung, beispielsweise einem Magnetschloss, geschlossen. Dadurch ist der Bettgurt an dem Bett befestigt. An dem Bettgurt ist ein Leibgurt befestigt, der ringförmig um die Taille des Patienten herumgelegt und mit einer weiteren Verriegelung geschlossen werden kann. Dadurch ist der Patient an der Taille auf dem Bett fixiert. Dies schützt den Patienten vor einem Herausfallen aus dem Bett, ohne seine Bewegungsfreiheit insbesondere an den Händen und Füßen unnötig einzuschränken. Eine derartige Fixierbandage ist in dem Gebrauchsmuster DE 203 17 701 U1 beschrieben.

Um eine bessere Fixierung des Patienten zu erzielen, ist aus dem Gebrauchsmuster DE 20 2006 009 390 U1 eine Fixierbandage mit einem zusätzlichen Fixiergurt bekannt, der an dem Leibgurt und seitlich an den Betthalterungen befestigt wird.

Die in dem Gebrauchsmuster DE 203 17 701 U1 beschriebene Fixierbandage hat zur zusätzlichen seitlichen Befestigung zwei fest mit dem Leibgurt verbundene Seitenbefestigungen, deren freie Enden an den Betthalterungen verriegelt werden. Diese Seitenbefestigungen können zuverlässig verhindern, dass der Patient sich auf die Seite dreht. Dies ist in vielen Fällen aus Sicherheitsgründen unbedingt erforderlich, um ein seitliches Herauslehnen oder Herausrutschen des Oberkörpers aus dem Bett zu verhindern, wobei es zu Verletzungen durch den Leibgurt kommen kann.

Aus der Druckschrift JP 60 055944 A ist eine Fixierbandage zur Fixierung der Oberschenkel eines Patienten bekannt geworden. Diese weist zwei jeweils um einen Oberschenkel herum geschlossene Gurte auf. An jeder Seite der Fixierbandage ist eine Betthalterung angeordnet, die durch eine an der Oberseite des betreffenden Oberschenkels angeordnete Schlaufe geführt wird. Ausgehend von diesen Schlaufen werden die Betthalterungen jeweils um einen Bettholm herum und dann zur Mitte Zurückgeführt, wo die freien Enden der Betthalterungen oberhalb der Oberschenkel miteinander verbunden werden.

Davon ausgehend ist es die Aufgabe der Erfindung, eine Fixierbandage zur Verfügung zu stellen, die in der Praxis eine sicherere Fixierung eines Patienten ermöglicht.

Diese Aufgabe wird gelöst durch die Fixierbandage zur Fixierung eines Patienten auf einem Bett oder einem sonstigen Lager mit den Merkmalen des Anspruchs 1. Vorteilhafte Ausgestaltungen sind in den sich anschließenden Unteransprüchen angeweben.

Die Fixierbandage hat
- einen ringförmig um die Taille des Patienten zur schließenden Leibgurt,
- einen flach auf eine Liegefläche des Betts aufzulegenden Bettgurt, der zur Befestigung an einem Bettgestell des Betts an jedem seiner beiden Enden eine Betthalterung aufweist und an dem der Leibgurt befestigt ist, wobei im Gebrauch der Fixierbandage Abschnitte des Bettgurts zwischen dem Leibgurt und einem Holm eines Bettgestell auf der Liegefläche aufliegen und
- zur zusätzlichen seitlichen Befestigung des Leibgurts die Betthalterungen jeweils von einem Befestigungsgurt gebildet sind, dessen Länge so bemessen ist, dass er ausgehend von einem der Enden des Bettgurts um einen Holm des Bettgestells herumgeführt und zu dem Leibgurt zurückgeführt werden kann, und der zur Befestigung an dem Leibgurt ausgebildet ist.

Die Erfindung beruht auf der Erkenntnis, dass die eingangs geschilderten bekannten Fixierbandagen zwar grundsätzlich eine schere Fixierung ermöglichen, in der Praxis jedoch nicht immer korrekt angelegt werden. Der Grund hierfür ist, dass durch die Fixierung des Bettgurts und das Schließen des Leibgurts um den Patienten herum bereits eine gewisse Fixierung des Patienten erreicht wird. Für eine vollständige und hinreichend sichere Fixierung ist jedoch zwingend ein zusätzliches Befestigen der Seitenbefestigungen notwendig. Dies kann bei der bekannten Fixierbandage jedoch im alltäglichen Gebrauch wegen Überlastung im Pflegebereich unterlassen werden.

Die Erfindung schafft hier Abhilfe, indem die Betthalterungen als Befestigungsgurte ausgeführt sind, die zusätzlich zur Fixierung des Bettgurts am Bett zur seitlichen Befestigung des Leibgurts dienen. Hierzu wird jeder der beiden Befestigungsgurte um einen Holm des Bettgestells herumgeführt und an dem Leibgurt befestigt. Da in der Regel erst durch diese Befestigung eine Fixierung der Fixierbandage an dem Bett erfolgt, besteht nicht länger die Gefahr, dass eine Befestigung herkömmlicher, gesonderter Seitenbefestigungen unterbleibt. Mit dem Befestigen der Befestigungsgurtc am Leibgurt erfolgt gleichzeitig eine Befestigung des Bettgurts am Bettgestell und die seitliche Befestigung. Insbesondere ist bei der Erfindung zusätzlich zu dem Herumführen der Befestigungsgurte um die Holme des Bettgestells und der Befestigung der Befestigungsgurte an dem Leibgurt keine gesonderte Befestigung der Fixierbandage an dem Bettgestell erforderlich. Es kommt daher in der Regel erst durch das Befestigen der Befestigungsgurte am Leibgurt zu einer Fixierung der gesamten Anordnung an dem Bett.

Ein weiterer Vorteil der Erfindung ist, dass eine bestehende Fixierbandage, die noch keine oder nur herkömmliche, gesonderte Seitenbefestigungen aufweist, einfach gemäß der Erfindung nachgerüstet werden kann. Hierzu kann die bestehende Fixierbandage mit erfindungsgemäß ausgebildeten Betthalterungen ausgerüstet werden.

Die erfindungsgemäße Fixierbandage dient zur Fixierung eines Patienten auf einem Bett oder einem sonstigen Lager. Mit Fixierung ist einerseits eine Ruhigstellung verwirrter, unkooperativer oder nicht zurechnungsfähiger Patienten gemeint. Einbezogen ist darüber hinaus eine Lagerung von Patienten in einer bevorzugten Haltung aus anderen Gründen, beispielsweise zur Decubitus-Prophylaxe. Das sonstige Lager kann beispielsweise eine Trage oder eine Liege sein, beispielsweise für einen Krankentransport. Soweit in den Anspruchsmerkmalen auf Elemente des Betts wie das Bettgestell oder einen Holm des Bettgestells Bezug genommen wird, geschicht dies der Einfachheit halber stellvertretend für entsprechende Komponenten anderer Lager, Beispielsweise kann das Bettgestell im Falle einer Trage ein Rahmen der Trage sein und der Holm des Bettgestells ein seitlicher Träger dieses Rahmens.

Der Bettgurt wird flach auf eine Liegefläche des Betts, beispielsweise auf eine Matratze, aufgelegt. An beiden Enden des Bettgurts schließen sich Betthalterungen an, die von Abschnitten der Befestigungsgurte gebildet werden. Ein weiterer Abschnitt jedes Befestigungsgurts auf jeder Seite bildet eine Seitenbefestigung.

Der Leibgurt umschließt im Gebrauch die Taille des Patienten. Er kann relativ breit und gegebenenfalls gepolstert ausgeführt sein, um einen hohen Tragekomfort zu bieten. Er kann eine Einstellmöglichkeit aufweisen, beispielsweise in Form von in Umfangsrichtung beabstandeten Ösen, durch die eine Verriegelung hindurchgeführt werden kann. Dadurch kann der Leibgurt stets ausreichend fest angelegt werden, ohne zu unbequem zu sein.

Der Leibgurt ist an dem Bettgurt befestigt. Er kann beispielsweise unmittelbar mit dem Bettgurt vernäht sein, insbesondere entlang einer quer zur Längsrichtung der Gurte verlaufenden Mittellinie des Bettgurts und des Leibgurts. Die Verbindung kann sich auch über einen Längsabschnitt des Leibgurts und des Bettgurts erstrecken. Die Befestigung des Leibgurts an dem Bettgurt kann auch mit Hilfe von weiteren Gurten erfolgen, die sich über einen Längsabschnitt des Leibgurts und des Bettgurts erstrecken und im Wesentlichen an ihren Enden mit jeweils einem der beiden Gurte verbunden sind. Derartige Gurte können kreuzweise angeordnet sein, sodass der ringförmig geschlossene Leibgurt ein Stück weit auf dem Bettgurt abgerollt werden kann, um eine begrenzte Drehbewegung des Patienten zuzulassen.

Jeder der beiden Befestigungsgurte ersteckt sich von einem Ende des Bettgurts im Gebrauch zunächst in seitlicher Richtung zu einem Holm des Bettgestells (oder, beispielsweise, einem seitlichen Träger eines Rahmens einer Trage) hin, um den Holm herum und zurück zu dem Leibgurt. Er ist zur Befestigung an dem Leibgurt ausgebildet, d.h. er weist beispielsweise weitere hierfür geeignete Ösen auf, die mit einer Verriegelung an dem Leibgurt befestigt werden. Die Befestigung der Befestigungsgurte an dem Leibgurt erfolgt insbesondere an Abschnitten des Leibgurts, die im Gebrauch deutlich oberhalb der Liegefläche angeordnet sind, beispielsweise zwischen den im Gebrauch seitlich am weitesten außen angeordneten Positionen des geschlossenen Leibgurts an dessen Oberseite.

In einer Ausgestaltung sind der Bettgurt und die beiden Befestigungsgurte von einem einzigen Gurt gebildet. Grundsätzlich kann der Bettgurt ein anderer, mit dem Befestigungsgurt verbundener Gurt sein. Der Bettgurt, der sich im Gebrauch unterhalb des Patienten befindet, kann auch eine größere Breite aufweisen als die Befestigungsgurte, um ein Verdrehen des Bettgurts zu verhindern. Die Befestigungsgurte können mit dem Bettgurt vernäht sein. Gemäß der Ausgestaltung sind Bettgurt und Befestigungsgurt von einem einzigen Gurt gebildet, sodass keine Verbindung von die Befestigungsgurte und den Bettgurt bildenden Abschnitten vorhanden ist. Dies ermöglicht eine besonders einfache Fertigung der Fixierbandage. Wahlweise kann im Bereich des den Bettgurt bildenden Abschnitts des einzigen Gurts eine Verbreiterung oder ein Polster vorgesehen sein. Dies ist jedoch nicht erforderlich, da der Gurt in der Regel auf der ohnehin gepolsterten Liegefläche aufliegt.

In einer Ausgestaltung ist zum Schließen des Leibgurts und zum Befestigen der Befestigungsgurte an dem Leibgurt eine einzige Verriegelung vorgesehen. Grundsätzlich können bei der Erfindung zum Schließen des Leibgurts und zum Befestigen der beiden Bettgurte an dem Leibgurt jeweils getrennte Verriegelungen verwendet werden, sodass beispielsweise drei Verriegelungen erforderlich sind, um die Fixierbandage ordnungsgemäß anzulegen. Es können jedoch auch beide Befestigungsgurte mit einer gemeinsamen Verriegelung an dem Leibgurt befestigt werden, wobei zum Schließen des Leibgurts weiterhin eine gesonderte Verriegelung zum Einsatz kommt. Bevorzugt wird jedoch nur eine einzige Verriegelung verwendet, die dann gleichzeitig den Leibgurt schließt und die beiden Befestigungsgurte an dem Leibgurt befestigt. Auf diese Weise wird eine besonders kostengünstige und materialsparende Fixierbandage zur Verfügung gestellt. Es ist nur eine einzige Verriegelung, beispielsweise ein Magnetschloss, erforderlich. Auch die Gurte können einfacher ausgeführt werden, weil beispielsweise gesonderte Ösen zur Aufnahme der weiteren Verriegelungen nicht benötigt werden. Zudem wird das Anlegen der Fixierbandage und insbesondere auch das Öffnen derselben vereinfacht, weil nur eine einzige Verriegelung geschlossen bzw. geöffnet werden muss.

In einer Ausgestaltung ist die Verriegelung ein Magnetschloss. Die Verwendung von Magnetschlössern zur Verriegelung von Fixierbandagen ist bekannt. Sie stellt eine besonders einfach zu handhabende und dennoch sichere Verriegelungslösung dar. Das Magnetschloss kann in der Regel ohne spezielles Werkzeug durch einfaches Zusammenstecken geschlossen werden. Öffnen lässt es sich ebenfalls sehr einfach mit Hilfe eines Magnetschlüssels.

In einer Ausgestaltung weist das Magnetschloss einen Schließknopf und einen Sockel mit einem Teller und einem Schaft auf, wobei die Länge des Schafts so bemessen ist, dass er durch zwei Ösen des Leibgurts und jeweils eine Öse jedes Befestigungsgurts hindurchführbar und der Schließknopf oberhalb der Gurte auf den Schaft aufsteckbar ist. Der Teller des Sockels ist eine beispielsweise kreisrunde oder rechteckige Grundplatte, die in der Regel unterhalb des untersten Gurts angeordnet wird. Durch einen relativ großflächigen Teller wird einer Verletzungsgefahr entgegengewirkt. Der Teller kann beispielsweise durch eine an der Unterseite des untersten Gurts aufgenähte Tasche an dem untersten Gurt gehalten werden. Der Schaft wird dann seitlich in die Tasche hinein und durch eine Öse des untersten Gurts hindurchgeführt, bis der Teller an dem Gurt anliegt. Durch die Tasche ist der Sockel gegen ein einfaches Herausrutschen aus der Öse gesichert. Der unterste Gurt ist bevorzugt ein Ende des Leibgurts. Die im Gebrauch darüberliegende Gurtlage kann insbesondere das andere Ende des Leibgurts sein, der ebenfalls mindestens eine Öse aufweist, durch die der Schaft hindurchgeführt wird. Anschließend wird der Schaft durch jeweils eine Öse jedes der beiden Befestigungsgurte hindurchgeführt bzw. Öffnungen der Gurte werden auf den Schaft aufgesteckt. Durch die an die insgesamt vier Lagen der unterschiedlichen Gurte angepasste Länge des Schafts steht dessen von dem Teller entferntes Ende anschließend aus dem obersten Gurt hervor, sodass der Schließknopf aufgesetzt werden kann. Insgesamt wird auf einfache Weise mit einem einzigen Magnetschloss ein ordnungsgemäßes Anlegen der Fixierbandage und eine zuverlässige und sichere Fixierung des Patienten erreicht.

In einer Ausgestaltung ist an den im Gebrauch der Fixierbandage zwischen einem der Holme des Bettgestells und dem Leibgurt auf der Liegefläche aufliegenden Aschnitten der Befestigungsgurte der des Bettgurts jeweils ein Ring oder eine Schlaufe angeordnet, durch den oder die der Befestigungsgurt nach dem Herumführen um den Holm des Bettgestells hindurchführbar ist, sodass er nahe an der Liegefläche gehalten wird. Der Befestigungsgurt kann in dem Ring oder in der Schlaufe frei verschieblich sein. Der Ring kann beispielsweise ein rechteckiger Metallring sein. Die Schlaufe kann beispielsweise von einem aufgenähten Stück Gurtmaterial gebildet sein. In beiden Fällen wird durch das Hindurchführen des Befestigungsgurts derselbe zur Liegefläche hin heruntergezogen, wodurch ein größerer Freiraum unterhalb dem die Seitenbefestigung bildenden Abschnitt des Befestigungsgurts vermieden wird. Dadurch wird verhindert, dass der Patient mit einem Körperteil, insbesondere mit dem Kopf oder Hals, zwischen die genannten Gurtabschnitte geraten kann. Einer Verletzungsgefahr wird auf diese Weise mit einfachen Mitteln entgegengewirkt.

In einer Ausgestaltung ist an im Gebrauch der Fixierbandage seitlich der Taille angeordneten Abschnitten des Leibgurts jeweils ein Ring oder eine Schlaufe angeordnet, durch die jeweils einer der Befestigungsgurte hindurchführbar ist, sodass die Befestigungsgurte von den Ringen oder den Schlaufen nahe an dem Leibgurt gehalten werden. Die Ringe oder Schlaufen können wie vorstehend beschrieben ausgeführt sein. Auch bei dieser Ausgestaltung wird erreicht, dass ein Freiraum geschlossen wird, in diesem Fall zwischen den die Seitenbefestigungen bildenden Abschnitten der Befestigungsgurte und dem Leibgurt, hier durch Heranziehen der Befestigungsgurte an den Leibgurt. Die Sicherheit der Fixierbandage wird dadurch weiter erhöht.

In einer Ausgestaltung sind an dem Bettgurt, an den genannten Abschnitten der Befestigungsgurte und/oder des Leibgurts mehrere voneinander beabstandete Ringe oder Schlaufen angeordnet, durch die der Befestigungsgurt nach dem Herumführen um den Holm des Bettgestells hindurchführbar ist. Beispielsweise können an den die Betthalterungen bildenden Abschnitten der Befestigungsgurte und/oder an dem Bettgurt jeweils zwei oder mehr Ringe oder Schlaufen angeordnet sein. Dadurch werden die die Seitenbefestigungen bildenden Abschnitte der Befestigungsgurte noch zuverlässiger nahe an der Liegefläche bzw. an dem Leibgurt geführt. Dies wirkt der erläuterten Verletzungsgefahr weiter entgegen.

In einer Ausgestaltung ist mindestens eine der Schlaufen schlauchförmig ausgebildet. In diesem Fall werden die die Seitenbefestigungen bildenden Abschnitte der Befestigungsgurte durch die schlauchförmigen Schlaufen hindurchgeführt, sodass sie in einem relativ langen Längsabschnitt geführt sind. Auch dies wirkt der erläuterten Verletzungsgefahr weiter entgegen.

In einer Ausgestaltung weist der Leibgurt einen Klettverschluss auf, mit dem die Enden des Leibgurts ringförmig verbindbar sind. Dies ermöglicht eine vorläufige Fixierung des Leibgurts in einem um die Taille geschlossenen Zustand und ist insbesondere dann sehr hilfreich, wenn der Leibgurt mit derselben Verriegelung geschlosscn werden soll, mit der auch die Befestigungsgurte an dem Leibgurt befestigt werden sollen. Ein unbeabsichtigtes Verrutschen des Leibgurts während des Anlegens der Befestigungsgurte und vor der endgültigen Verriegelung wird dadurch vermieden.

In einer Ausgestaltung sind an einer Außenseite des Leibgurts und an der der Außenseite im Gebrauch zugewandten Seite der Befestigungsgurte Klettverschlusselemente angeordnet, die eine vorläufige Fixierung der Befestigungsgurte an dem Leibgurt ermöglichen. Die Klettverschlusselemente sind insbesondere ein Klettverschlussflausch und ein Klettverschlusshaken. Diese Ausgestaltung vereinfacht das Anliegen der Fixierbandage, weil ein Verrutschen der Befestigungsgurte bis zum Schließen der Verriegelung verhindert wird.

In einer Ausgestaltung weist die Fixierbandage einen Schrittgurt oder eine Oberschenkelhalterung und/oder eine Schulterhalterung auf. Durch die zusätzlichen Elemente wird insbesondere ein Herausrutschen des Patienten aus dem Leibgurt nach oben oder unten verhindert.

In einer Ausgestaltung weist die Fixierbandage Handmanschetten oder Schlaufen zur Befestigung von Handmanschetten auf. Die Handmanschetten oder Schlaufen können insbesondere an dem Bettgurt oder an den Befestigungsgurten angeordnet und befestigt sein. Sie ermöglichen eine besonders einfache zusätzliche Fixierung der Hände bzw. Arme des Patienten.

Die Erfindung wird nachfolgend anhand von in zwei Figuren dargestellten Ausführungsbeispielen näher erläutert. Es zeigen:
- Fig. 1: eine erfindungsgemäße Fixierbandage im angelegten Zustand in einer vereinfachten Querschnittsdarstellung und
- Fig. 2: eine Alternative eines in Figur 1 dargestellten Details.

Es werden in beiden Figuren für sich entsprechende Teile die gleichen Bezugszeichen verwendet.

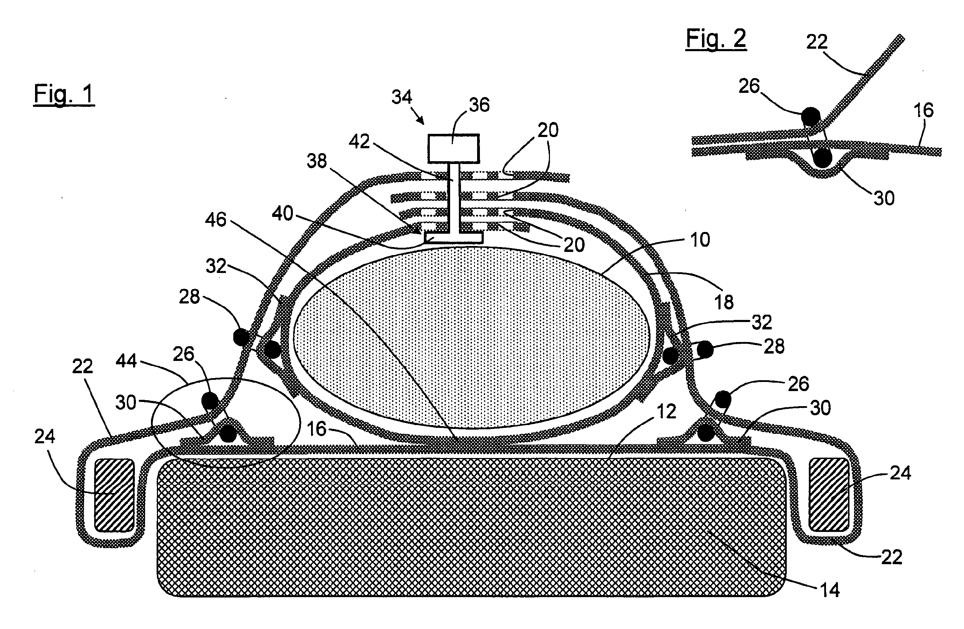

Fig. 1 zeigt die Taille 10 eines Patienten, der auf einer Liegefläche 12 einer Matratze 14 eines Betts liegt. Der Patient ist mit einer erfindungsgemäßen Fixierbandage fixiert, die einen auf der Liegefläche 12 aufliegenden Bettgurt 16 aufweist, der wie die übrigen Gurte auch aus einem textilen Gurtmaterial besteht. Der Bettgurt 16 erstreckt sich annähernd über die gesamte Breite der Liegefläche 12 und weist zwei Enden auf, die sich nahe den Kanten der Matratze 14 befinden.

In der Mitte des Bettgurts 16 ist bei 46 ein Leibgurt 18 befestigt, der die Taille 10 des Patienten ringförmig umschließt. Der Leibgurt 18 ist ein Köfperteilgurt für den Körperteil "Taille" des Patienten. An beiden Enden des Leibgurts 18 sind jeweils mehrere Ösen 20 angeordnet, wobei im ringförmig geschlossenen Zustand des Leibgurts 18 mindestens zwei der Ösen 20 übereinander liegen.

Die Fixierbandage ist mit einem einzigen Magnetschloss 34 geschlossen. Das Magnetschloss 34 weist einen Schließknopf 36 und einen Sockel 38 mit einem Teller 40 und einem Schaft 42 auf. Der Teller 40 befindet sich unterhalb des unteren Endes des Leibgurts 18 und der Schaft 42 ist durch eine Öse 20 jeder der vier Gurtlagen hindurchgeführt. Oberhalb der obersten Gurtlage, die im Beispiel von dem in der Figur links angeordneten Befestigungsgurt 22 gebildet wird, ist der Schließknopf 36 aufgesteckt.

An jedes Ende des Bettgurts 16 schließt sich jeweils ein Befestigungsgurt 22 an, der ausgehend von dem jeweiligen Ende des Bettgurts 16 um einen Holm 24 des Bettgestells herumgeführt ist und von dort zu dem Leibgurt 18 zurückgeführt ist. Genauer erstreckt sich der Befestigungsgurt 22 von dem Holm 24 ausgehend durch zwei rechteckige Ringe 26, 28 aus Metall hindurch und von dort weiter parallel zum Leibgurt 18 bis zu einer Oberseite des Leibgurts 18, wo die beiden Enden des Leibgurts 18 mit den Ösen 20 übereinanderliegen. Auch das freie Ende jedes Befestigungsgurts 22 weist Ösen 20 auf, von denen jeweils mindestens eine oberhalb einer Öse 20 des Leibgurts angeordnet ist.

Die Ringe 26 sind jeweils mit einem Stück Gurtmaterial 30 an der Oberseite des Bettgurts 16 befestigt, und zwar in einem im Gebrauch seitlich zwischen dem Leibgurt 18 und dem Holm 24 des Bettgestells befindlichen Abschnitt. Die beiden Ringe 28 sind jeweils an einer Seite des Leibgurts 18 befestigt, ebenfalls mit einem Stück Gurtmaterial 32.

Figur 2 zeigt eine Alternative für den in Figur 1 mit 44 bezeichneten Ausschnitt. Gezeigt ist ebenfalls ein Ring 26, der an dem Bettgurt 16 befestigt und durch den ein Befestigungsgurt 22 hindurchgeführt ist. Im Unterschied zu Figur 1 verläuft bei der in Figur 2 dargestellten Alternative auch der Bettgurt 16 selbst durch den Ring hindurch und ein aufgenähtes Stück Gurtmaterial 30 befmdet sich an der Unterseite des Bettgurts 16. Es dient der Fixierung des Rings 26 an der gewünschten Position des Bettgurts 16.

### Liste der verwendeten Bezugszeichen:

10 Taille
12 Liegefläche
14 Matratze
16 Bettgurt
18 Leibgurt
20 Öse
22 Befestigungsgurt
24 Holm
26 Ring (am Bettgurt)
28 Ring (am Leibgurt)
30 Gurtmaterial zur Befestigung der Ringe 26 am Bettgurt
32 Gurtmaterial zur Befestigung der Ringe 28 am Leibgurt
34 Magnetschloss
36 Schließknopf
38 Sockel
40 Teller
42 Schaft
44 Ausschnitt aus Figur 1
46 Befestigungsbereich Bettgurt/Leibgurt

## Patentansprüche

1. Fixierbandage zur Fixierung eines Patienten auf einem Bett oder einem sonstigen Lager mit
• einem ringförmig um die Taille des Patienten zu schließenden Leibgurt (18),
• einem flach auf eine Liegefläche (12) des Betts aufzulegenden Bettgurt (16), der zur Befestigung an einem Bettgestell des Betts an jedem seiner beiden Enden eine Betthalterung aufweist und an dem der Leibgurt (18) befestigt ist, wobei im Gebrauch der Fixierbandage Abschnitte des Bettgurts (16) zwischen dem Leibgurt (18) und einem Holm (24) eines Bettgestells auf der Liegefläche (12) aufliegen, **dadurch gekennzeichnet, dass**
• zur zusätzlichen seitlichen Befestigung des Leibgurts (18) die Betthalterungen jeweils von einem Befestigungsgurt (22) gebildet sind, dessen Länge so bemessen ist, dass er ausgehend von einem der Enden des Bettgurts (16) um einen Holm (24) des Bettgestells herumgeführt und zu dem Leibgurt (18) zurückgeführt werden kann, und der zur Befestigung an dem Leibgurt (18) ausgebildet ist.

2. Fixierbandage nach Anspruch 1, **dadurch gekennzeichnet, dass** der Bettgurt (16) und die beiden Befestigungsgurte (22) von einem einzigen Gurt gebildet sind.

3. Fixierbandage nach Anspruch 1 oder 2, **dadurch gekennzeichnet, dass** zum Schließen des Leibgurts (18) und zum Befestigen der Befestigungsgurte (22) an dem Leibgurt (18) eine einzige Verriegelung vorgesehen ist.

4. Fixierbandage nach Anspruch 3, dadurch gekenntzeichnet, dass die Verriegelung ein Magnetschloss (34) ist.

5. Fixierbandage nach Anspruch 4, **dadurch gekennzeichnet, dass** das Magnetschloss (34) einen Schließknopf(36) und einen Sockel (38) mit einem Teller (40) und einem Schaft (42) aufweist, wobei die Länge des Schafts (42) so bemessen ist, dass er durch zwei Ösen (20) des Leibgurts (18) und jeweils eine Öse (20) jedes Befestigungsgurts (22) hindurchführbar und der Schließknopf (36) oberhalb der Gurte auf den Schaft (42) aufsteckbar ist.

6. Fixierbandage nach einem der Ansprüche 1 bis 5, dadurch gekennzeichet, dass an den im Gebrauch der Fixierbandage zwischen einem der Holme (24) des Bettgestells und dem Körperteilgurt auf der Liegefläche (12) aufliegenden Abschnitten der Befestigungsgurte (22) oder des Bettgurts (16) jeweils ein Ring (26) oder eine Schlaufe angeordnet ist, durch den oder die der Befestigungsgurt (22) nach dem Herumführen um den Holm (24) des Bettgestells hindurchführbar ist, so dass er nahe an der Liegefläche (12) gehalten wird.

7. Fixierbandage nach einem der Ansprüche 1 bis 6, dadurch gekenntzeichnet, dass an im Gebrauch der Fixierbandage seitlich der Taille angeordneten Abschnitten des Leibgurts (18) jeweils ein Ring (28) oder eine Schlaufe angeordnet ist, durch die jeweils einer der Befestigungsgurte (22) hindurchführbar ist, so dass die Befestigungsgurte (22) von den Ringen oder den Schlaufen nahe an dem Leibgurt (18) gehalten werden.

8. Fixierbandage nach Anspruch 6 oder 7, **dadurch gekennzeichnet, dass** an dem Bettgurt (16), an den Abschnitten der Befestigungsgurte (22) und/oder des Leibgurts (18) mehrere voneinander beabstandete Ringe (26, 28) oder Schlaufen angeordnet sind, durch die der Befestigungsgurt (22) nach dem Herumführen um den Holm (24) des Bettgestells hindurchführbar ist.

9. Fixierbandage nach einem der Ansprüche 6 bis 8, **dadurch gekennzeichnet, dass** mindestens eine der Schlaufen schlauchförmig ausgebildet ist.

10. Fixierbandage nach einem der Ansprüche 1 bis 9, **dadurch gekennzeichnet, dass** der Leibgurt (18) einen Klettverschluss aufweist, mit dem die Enden des Leibgurts (18) ringförmig werbindbar sind.

11. Fixierbandage nach einem der Ansprüche 1 bis 10, **dadurch gekennzeichnet, dass** an einer Außenseite des Leibgurts (18) und an den der Außenseite im Gebrauch zugewandten Seiten der Befestigungsgurte (22) Klettverschlusselemente angeordnet sind, die eine vorläufige Fixierung der Befestigungsgurte (22) an dem Leibgurt (18) ermöglichen.

12. Fixierbandage nach einem der Ansprüche 1 bis 11, **dadurch gekennzeichnet, dass** die Fixierbandage einen Schrittgurt oder eine Oberschenkelhalterung und/oder eine Schulterhalterung aufweist.

13. Fixierbandage nach einem der Ansprüche 1 bis 12, **dadurch gekennzeichnet, dass** die Fixierbandage Handmanschetten oder Schlaufen zur Befestigung von Handmanschetten aufweist.

## Claims

1. A fixation bandage for fixing a patient on a bed or another repository, with
• a waist belt (18) to be closed around the waist of the patient in a ring-shaped fashion,
• a bed belt (16), flatly to be put onto a reclining surface (12) of the bed and having a bed holder on each of its two ends for fastening it on a bed frame of the bed, and on which the waist belt (18) is fastened, wherein in use of the fixation bandage, sections of the bed belt (16) are lying on the reclining surface (12) between the waist belt (18) and a beam (24) of a bed frame, **characterised in that**
• for additional lateral fastening of the waist belt (18), the bed holders are each one formed by one fastening belt (22), whose length is dimensioned such that starting from one of the ends of the bed belt (16), it can be guided around a beam (24) of the bed frame and can be guided back to the waist belt (18) and is adapted to be fastened on the waist belt (18).

2. The fixation bandage according to claim 1, **characterised in that** the bed belt (16) and the two fastening belts (22) are formed by one single belt.

3. A fixation bandage according to claim 1 or 2, **characterised in that** one single locking device is provided for closing the waist belt (18) and for fastening the fastening belts (22) on the waist belt (18).

4. The fixation bandage according to claim 3, **characterised in that** the locking device is a magnetic lock (34).

5. The fixation bandage according to claim 4, **characterised in that** the magnetic lock (34) has a locking button (36) and a base (38) with a disc (40) and a shaft (42), wherein the length of the shaft (42) is dimensioned such that it can be guided through two ears (20) of the waist belt (18) and through one ear (20) of each fastening belt (22), and the locking button (36) can be put onto the shaft (42) above the belts.

6. A fixation bandage according to any one of claims 1 to 5, **characterised in that** on each of the sections of the fastening belts (22) or of the bed belt (16) that lie on the reclining surface (12) between one of the beams (24) of the bed frame and the waist belt (18) in use of the fixation bandage, one ring (26) or one loop is arranged, through which the fastening belt (22) can be guided after having been guided around the beam (24) of the bed frame, so that it is held near to the reclining surface (12).

7. A fixation bandage according to any one of claims 1 to 6, **characterised in that** on each of the sections of the of the waist belt (18) that are arranged at the side of the waist in use of the fixation bandage, one ring (28) or one loop is arranged through which one of the fastening belts (22) can be guided through, so that the fastening belts (22) are held near to the waist belt (18) by the rings or loops.

8. A fixation bandage according to claim 6 or 7, **characterised in that** plural spaced apart rings (26, 28) or loops are arranged on the bed belt (16),on the sections of the fastening belts (22) and/or of the waist belt (18) through which the fastening belt (22) can be guided after having been guided around the beam (24) of the bed frame.

9. A fixation bandage according to any one of claims 6 to 8, **characterised in that** at least one of the loops is formed in the form of a hose.

10. A fixation bandage according to any one of claims 1 to 9, **characterised in that** the waist belt (18) has a Velcro fastener by which the ends of the waist belt (18) can be connected in a ring shaped fashion.

11. A fixation bandage according to any one of claims 1 to 10, **characterised in that** Velcro fastener elements permitting a preliminary fixation of the fastening belts (22) on the waist belt (18) are arranged on an outer side of the waist belt (18) and on those sides of the fastening belts (22) that in use face the outer side.

12. A fixation bandage according to any one of claims 1 to 11, **characterised in that** the fixation bandage comprises a crotch strap or a thigh holder and/or a shoulder holder.

13. A fixation bandage according to any one of claims 1 to 12, **characterised in that** the fixation bandage has wrist cuffs or loops for fastening wrist cuffs.

## Revendications

1. Bandage fixe pour la fixation d'un patient sur un lit ou sur un autre support, avec
• une sangle abdominale (18) à fermer de façon annulaire autour de la taille du patient,
• une sangle de lit (16), à placer à plat sur un plan de couchage (12) du lit, qui présente, pour la fixation sur un bâti de lit du lit, à chacune de ses deux extrémités, une retenue de lit et sur laquelle est fixée la sangle abdominale (18), des tronçons de la sangle de lit (16) reposant, pendant l'utilisation du bandage fixe, sur le plan de couchage (12) entre la sangle abdominale (18) et un longeron (24) d'un bâti de lit, **caractérisé en ce que**
• pour la fixation latérale supplémentaire de la sangle abdominale (18), les retenues de lit sont formées respectivement d'une sangle de fixation (22) dont la longueur est dimensionnée de telle sorte que, en partant d'une des extrémités de la sangle de lit (16), elle est conduite autour d'un longeron (24) du bâti de lit et elle peut être ramenée à la sangle abdominale (18), et qui est formée pour la fixation sur la sangle abdominale (18).

2. Bandage fixe selon la revendication 1, **caractérisé en ce que** la sangle de lit (16) et les deux sangles de fixation (22) sont formées d'une sangle unique.

3. Bandage fixe selon la revendication 1 ou 2, **caractérisé en ce que**, pour la fermeture de la sangle abdominale (18) et pour la fixation des sangles de fixation (22) sur la sangle abdominale (18), il est prévu un seul verrouillage.

4. Bandage fixe selon la revendication 3, **caractérisé en ce que** le verrouillage est une serrure magnétique (34).

5. Bandage fixe selon la revendication 4, **caractérisé en ce que** la serrure magnétique (34) présente un bouton de fermeture (36) et un socle (38) avec un plateau (40) et une tige (42), la longueur de la tige (42) étant dimensionnée de telle sorte qu'elle peut être introduite à travers deux oeillets (20) de la sangle abdominale (18) et respectivement à travers un oeillet (20) de chaque sangle de fixation (22), et de telle sorte que le bouton de fermeture (36) peut être enfiché au-dessus des sangles sur la tige (42).

6. Bandage fixe selon une des revendications 1 à 5, **caractérisé en ce que**, sur les tronçons des sangles de fixation (22) ou de la sangle de lit (16) reposant, pendant l'utilisation du bandage fixe, entre un des longerons (24) du bâti de lit et la sangle de partie corporelle, il est disposé respectivement un anneau (26) ou une boucle à travers lequel ou laquelle la sangle de fixation (22) peut être introduite après le passage autour du longeron (24) du bâti de lit, de telle sorte qu'elle est retenue à proximité sur plan de couchage (12).

7. Bandage fixe selon une des revendications 1 à 6, **caractérisé en ce que**, pendant l'utilisation du bandage fixe, sur des tronçons de la sangle abdominale (18) disposés latéralement par rapport à la taille, il est disposé respectivement un anneau (28) ou une boucle, à travers lequel ou laquelle respectivement une des sangles de fixation (22) peut être introduite, de telle sorte que les sangles de fixation (22) sont retenues à proximité de la sangle abdominale (18) par les anneaux ou les boucles.

8. Bandage fixe selon la revendication 6 ou 7, **caractérisé en ce que**, sur la sangle de lit (16), sur les tronçons des sangles de fixation (22) et/ou de la sangle abdominale (18), il est disposé plusieurs anneaux (26, 28) ou boucles espacé(e)s entre eux ou entre elles, à travers lesquels ou lesquelles la sangle de fixation (22) peut être introduite après avoir été passée autour du longeron (24) du bâti de lit.

9. Bandage fixe selon une des revendications 6 à 8, **caractérisé en ce qu'**au moins une des boucles est constituée en forme de tuyau.

10. Bandage fixe selon une des revendications 1 à 9, **caractérisé en ce que** la sangle abdominale (18) présente une fermeture auto-agrippante avec laquelle les extrémités de la sangle abdominale (18) peuvent être raccordées sous forme annulaire.

11. Bandage fixe selon une des revendications 1 à 10, **caractérisé en ce que**, sur un côté extérieur de la sangle abdominale (18) et sur les côtés des sangles de fixation (22) tournés vers le côté extérieur pendant l'utilisation, il est disposé des éléments de fermeture auto-agrippante qui permettent une fixation provisoire des sangles de fixation (22) sur la sangle abdominale (18).

12. Bandage fixe selon une des revendications 1 à 11, **caractérisé en ce que** le bandage fixe présente une sangle d'entrejambe ou une retenue des cuisses et/ou une retenue des épaules.

13. Bandage fixe selon une des revendications 1 à 12, **caractérisé en ce que** le bandage fixe présente des manchettes manuelles ou des boucles pour la fixation de manchettes manuelles.
